(19) Europäisches Patentamt
European Patent Office
Office européen des brevets

(11) Veröffentlichungsnummer: **0 497 300 A1**

# (12) EUROPÄISCHE PATENTANMELDUNG

(21) Anmeldenummer: **92101443.7**

(22) Anmeldetag: **29.01.92**

(51) Int. Cl.[5]: **C07D 313/00**, C07D 493/04, C07D 493/08, A61K 31/365, C12P 17/08, C12P 17/18

Der Anmelder hat eine Erklärung nach Regel 28 (4) EPÜ (Herausgabe einer Probe nur an einen Sachverständigen) eingereicht. Eingangsnummer(n) der Hinterlegung(en): DSM 4209 and DSM 4210.

(30) Priorität: **14.05.91 DE 4115674**
**30.01.91 DE 4102668**

(43) Veröffentlichungstag der Anmeldung: **05.08.92 Patentblatt 92/32**

(84) Benannte Vertragsstaaten: **AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Zeeck, Axel, Prof. Dr.**
**Brüder-Grimm-Allee 22**
**W-3400 Göttingen(DE)**
Erfinder: **Philipps, Siegrid, Dr.**
**Bultstrasse 12**
**W-3100 Celle(DE)**
Erfinder: **Mayer, Marion**
**Petrikirchstrasse 20**
**W-3400 Göttingen(DE)**
Erfinder: **Göhrt, Axel**
**Arndtstrasse 3**
**W-3400 Göttingen(DE)**
Erfinder: **Granzer, Ernold, Dr. Dr.**
**Falkensteiner Strasse 24**
**W-6233 Kelkheim (Taunus)(DE)**
Erfinder: **Hammann, Peter, Dr.**
**Bürgermeister-Rühl-Strasse 20**
**W-6223 Babenhausen(DE)**
Erfinder: **Kirsch, Reinhard, Dr.**
**Johannesallee 18**
**W-6230 Frankfurt am Main 80(DE)**
Erfinder: **Thiericke, Ralf, Dr.**
**Auestrasse 35**
**W-6057 Dietzenbach(DE)**

## (54) Neue Naturstoffe mit Zehnringlacton-Strukfur, Verfahren zu ihrer Herstellung und ihre Verwendung.

(57) Penicillium sp., insbesondere DSM 4209 und 4210, bildet unter spezifischen Fermentationsbedingungen neue Naturstoffe mit Zehnringlacton-Struktur der Formel I,

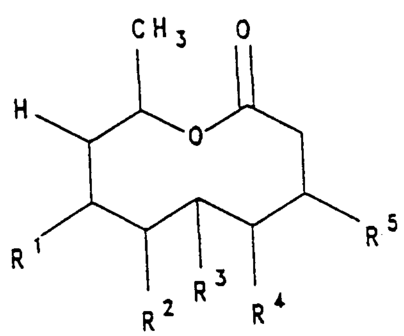

in der

EP 0 497 300 A1

$R^1$ Hydroxyl, Oxo oder zusammen mit $R^2$ eine Doppelbindung oder cyclisch mit Sauerstoff verknüpft ist, im letzteren Falle dann aber $R^3$ für Methoxy steht,

$R^2$ Wasserstoff oder zusammen mit $R^5$ cyclisch mit einem Sauerstoff verknüpft oder zusammen mit $R^3$ eine Doppelbindung bedeutet, im letzteren Falle dann aber $R^1$ für Oxo steht,

$R^3$ Wasserstoff, Hydroxy oder Methoxy oder zusammen mit $R^4$ eine Doppelbindung bedeutet,

$R^4$ Wasserstoff oder Hydroxy und

$R^5$ Wasserstoff oder Oxo bedeuten,

die antibakterielle und lipidregulatorische Eigenschaften besitzen und insbesondere die Biosynthese von Cholesterin beeinflussen.

Antibiotika aus Penicillium, wie z.B. Penicillin, sind schon lange bekannt und werden in großem Maßstab therapeutisch angewandt.

Es wurde gefunden, daß Penicillium-Stämme auch Stoffe ganz anderer Struktur synthetisieren können, nämlich Zehnringlactone (Decarestrictine). Die Verbindungen besitzen pharmakologische und damit therapeutische Wirksamkeit und haben insbesondere antibakterielle sowie lipidregulatorische Eigenschaften.

In der Patentanmeldung EP 333 024 sind Zehnringlactone beschrieben, welche bevorzugt von Penicillium DSM 4209 und DSM 4210 gebildet werden. Bei intensiver Untersuchung dieser Stämme wurde gefunden, daß in Abhängigkeit von den Fermentationsbedingungen überraschenderweise weitere Derivate mit ähnlichen Strukturen gebildet werden, welche deutliche antibakterielle sowie lipidregulatorische Eigenschaften besitzen und insbesondere die Biosynthese von Cholesterin beeinflussen.

Die Erfindung betrifft somit

1. ein Verfahren zur Herstellung der Verbindungen der Formel I

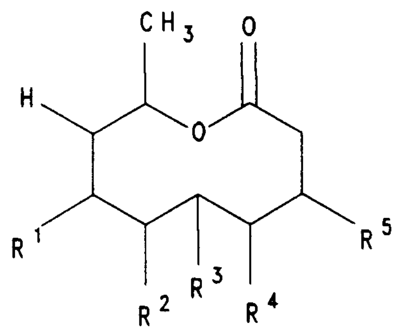

in der

- $R^1$ Hydroxyl, Oxo oder zusammen mit $R^2$ eine Doppelbindung oder cyclisch mit Sauerstoff verknüpft ist, im letzteren Falle dann aber $R^3$ für Methoxy steht,
- $R^2$ Wasserstoff oder zusammen mit $R^5$ cyclisch mit Sauerstoff verknüpft oder zusammen mit $R^3$ eine Doppelbindung bedeutet, im letzteren Falle dann aber $R^1$ für Oxo steht,
- $R^3$ Wasserstoff, Hydroxy oder Methoxy oder zusammen mit $R^4$ eine Doppelbindung bedeutet,
- $R^4$ Wasserstoff oder Hydroxy und
- $R^5$ Wasserstoff oder Oxo bedeuten,

das dadurch gekennzeichnet ist, daß Penicillium sp., insbesondere DSM 4209 oder DSM 4210, in einem Nährmedium kultiviert wird, bis sich die Verbindung der Formel I in der Kultur anhäuft,

2. eine Verwendung der Verbindungen der Formel I als Pharmazeutika, insbesondere als antibakterielles Mittel sowie zur Regulation des Lipidstoffwechsels.

Im folgenden wird die Erfindung detailliert beschrieben, insbesondere in ihren bevorzugten Ausführungsformen. Ferner wird die Erfindung in den Patentansprüchen definiert.

Die Verbindungen der Formel I werden bevorzugt aus Penicillium sp. DSM 4209 und DSM 4210 isoliert. Diese Stämme wurden aus einer Erdprobe aus dem Bryce Canyon, Utah, USA isoliert und bei der Deutschen Sammlung von Microorganismen nach den Regeln des Budapester Vertrages am 13. August 1987 unter den obengenannten Nummern hinterlegt. Konidien und Sporen des Pilzes wurden wie folgt charakterisiert:

| | |
|---|---|
| Konidien: | Monoverticilliata |
| Sporenoberfläche: | stachelig |
| Sporenfarbe: | graugrün |

In einer Nährlösung, die eine Kohlenstoffquelle und eine Stickstoffquelle sowie die üblichen anorganischen Salze enthält, synthetisiert Penicillium sp., bevorzugt DSM 4209 oder 4210, neben den in EP 333 024 genannten Verbindungen verschiedene Derivate der Formel I. Anstelle der Stämme DSM 4209 oder 4210 können natürlich auch deren Mutanten und Varianten eingesetzt werden, soweit sie Derivate der Formel I synthetisieren. Derartige Mutanten können in prinzipiell bekannter Weise durch physikalische Mittel, beispielsweise Bestrahlung wie mit UV- oder Röntgenstrahlen, oder durch chemische Mutagene, wie beispielsweise Ethylmethansulfonat (EMS), 2-Hydroxy-4-methoxy-benzophenon (MOB) oder N-Methyl-N'-nitro-N-nitrosoguanidin (MNNG), erzeugt werden.

Als bevorzugte Kohlenstoffquellen für die aerobe Fermentation eignen sich assimilierbare Kohlenhydrate und Zuckeralkohole, wie Glukose, Laktose oder D-Mannit sowie kohlenhydrathaltige Naturprodukte, wie z.B. Malzextrakt. Als stickstoffhaltige Nährstoffe kommen in Betracht: Aminosäuren, Peptide und Proteine sowie

deren Abbauprodukte, wie Peptone und Tryptone, ferner Fleischextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate. An anderen organischen Salzen kann die Nährlösung beispielsweise Chloride, Carbonate, Sulfate oder Phosphate der Alkali- oder Erdalkalimetalle, Eisen, Zink und Mangan enthalten.

Die Bildung der Verbindungen der Formel I verläuft besonders gut in einer Nährlösung, die etwa 0,2 bis 5 %, bevorzugt 1 bis 4 %, Malzextrakt, 0,02 bis 0,5 %, bevorzugt 0,1 bis 0,4 % Hefeextrakt, 0,1 bis 5 %, bevorzugt 0,5 bis 2 % Glucose und 0,005 bis 0,2 %, bevorzugt 0,01 bis 0,1 % Ammoniumsalze enthält, jeweils bezogen auf das Gewicht der gesamten Nährlösung. Die Kultivierung erfolgt aerob, also beispielsweise submers unter Schütteln oder Rühren in Schüttelkolben oder Fermentern, gegebenenfalls unter Einführen von Luft oder Sauerstoff. Sie kann in einem Temperaturbereich von etwa 18 bis 35 ° C, vorzugsweise bei etwa 25 bis 30 ° C, insbesondere bei 27 bis 28 ° C durchgeführt werden. Der pH-Bereich sollte zwischen 1 und 8 liegen, vorteilhaft zwischen 1 und 4. Man kultiviert den Mikroorganismus unter diesen Bedingungen im allgemeinen über einen Zeitraum von 60 bis 170 Stunden, bevorzugt 100 bis 150 Stunden.

Vorteilhaft kultiviert man in mehreren Stufen, d.h. man stellt zunächst eine oder mehrere Vorkulturen in einem flüssigen Nährmedium her, die dann in das eigentliche Produktionsmedium, die Hauptkultur, beispielsweise im Volumenverhältnis 1:10, überimpft werden. Die Vorkultur erhält man z.B., indem man ein versportes Mycel in eine Nährlösung überimpft und etwa 48 bis 72 Stunden wachsen läßt. Das versporte Mycel kann erhalten werden, indem man den Stamm etwa 7 Tage auf einem festen oder flüssigen Nährboden, beispielsweise Hefe-Malz-Agar, wachsen läßt.

Der Fermentationsverlauf kann anhand des pH-Wertes der Kultur oder des Mycelvolumens durch Dünnschichtchromatographie oder Ausprüfen der biologischen Aktivität überwacht werden. Die Verbindungen der Formel I sind sowohl im Mycel als auch im Kulturfiltrat enthalten.

Die Isolierung der genannten Verbindungen aus dem Kulturmedium erfolgt nach bekannten Methoden unter Berücksichtigung der chemischen, physikalischen und biologischen Eigenschaften der Produkte. Zum Testen der Antibiotika-Konzentration im Kulturmedium oder in den einzelnen Isolierungsstufen kann die Dünnschichtchromatographie, beispielsweise auf Kieselgel mit Chloroform/Methanol als Laufmittel, verwendet werden, wobei die Menge der gebildeten antibakteriellen Substanz zweckmäßig mit einer Eichlösung verglichen wird.

Zur Isolierung der Verbindungen werden Kulturbrühe und Mycel zuerst mit organischen Lösungsmitteln, wie z.B. Chloroform, Essigester usw. extrahiert, um die unpolaren Verunreinigungen zu entfernen. Anschließend wird mit einem stärker polaren Lösungsmittel, beispielsweise niederen Alkanolen oder Gemischen aus Chloroform und/oder Essigester mit einem niederen Alkanol, extrahiert.

Die Reinisolierung erfolgt vorzugsweise an geeigneten Medien, wie z.B. Kieselgel, Aluminiumoxid oder Ionenaustauschern, durch anschließende Elution mit organischen, polaren Lösungsmitteln oder Lösungsmittelgemischen, wie z.B. Essigester, Gemischen aus Essigester und einem niederen Alkanol gegebenenfalls mit Wasser, bzw. einem für Ionenaustauscher geeigneten Salzgradienten, wie beispielsweise Kochsalz oder Tris-(hydroxymethyl)aminomethan-HCl (Tris-Puffer), und Sammeln der wirksamen Fraktionen.

Die Verbindungen sind in festem Zustand und in Lösungen im pH-Bereich 2 bis 8, insbesondere 3 bis 7, stabil und lassen sich damit in übliche galenische Zubereitungen einarbeiten.

In den folgenden Beispielen wird die Erfindung in weiteren Details beschrieben. Prozentangaben beziehen sich auf das Gewicht, wenn nicht anders angegeben.

Beispiele:

1.

a) Herstellung einer Sporensuspension des Produzentenstammes: 100 ml Nährlösung (2 g Hefeextrakt; 20 g Malzextrakt, 10 g Glucose, 0,5 g $(NH_4)_2PO_4$, 1 l Leitungswasser, pH-Wert vor dem Sterilisieren 7,3) in einem 500 ml Erlenmeyerkolben werden mit dem Stamm DSM 4209 oder DSM 4210 beimpft und 72 Stunden bei 25 ° C und 120 UpM auf der rotierenden Schüttelmaschine inkubiert. Anschließend werden 20 ml Kulturflüssigkeit in einem 500 ml Erlenmeyerkolben mit dem Nährboden der obengenannten Zusammensetzung, dem 20 g Agar/l zur Verfestigung zugegeben wurde, gleichmäßig verteilt und dekantiert. Die Kulturen werden 10 bis 14 Tage bei 25 ° C inkubiert. Die nach dieser Zeit entstandenen Sporen eines Kolbens werden mit 500 ml entionisiertem Wasser, das einen Tropfen eines handelsüblichen nichtionischen Tensids (Triton X 100, Fa. Serva) enthält, abgeschwemmt, sofort weiterverwendet oder bei -22 ° C aufbewahrt.

b) Herstellung einer Kultur bzw. Vorkultur des Produzentenstammes im Erlenmeyerkolben.

Ein 500 ml Erlenmeyerkolben mit 100 ml der unter a) beschriebenen Nährlösung wird mit einer auf einem Schrägröhrchen gezogenen Kultur oder mit 0,2 ml Sporensuspension angeimpft und auf einer

Schüttelmaschine bei 120 UpM und 25 °C inkubiert. Die maximale Produktion der Verbindungen der Formel I ist nach ca. 120 Stunden erreicht. Zum Animpfen von 10 und 100 l Fermentern genügt eine 48 Stunden alte Submerskultur (5 %) aus der gleichen Nährlösung.

2. Herstellung der Verbindungen der Formel I

Ein 10 l Fermenter wird unter folgenden Bedingungen betrieben:

| | |
|---|---|
| Nährmedium: | 20,0 g/l Malzextrakt<br>2,0 g/l Hefeextrakt<br>10,0 g/l Glucose<br>0,5 g/l $(NH_4)_2PO_4$ |
| Inkubationszeit:<br>Inkubationstemperatur:<br>Rührgeschwindigkeit:<br>Belüftung: | 150 Stunden<br>25 °C<br>250 UpM<br>4 l Luft/min. |

Durch wiederholte Zugabe weniger Tropfen ethanolischer Polyollösung kann die Schaumentwicklung unterdrückt werden. Das Produktionsmaximum wird nach ca. 150 Stunden erreicht. Die Ausbeuten liegen zwischen 10 und 100 mg/l.

3. Isolierung der Verbindungen der Formel I

Nach der Fermentation von DSM 4209 bzw. DSM 4210 wird die Kulturbrühe unter Zusatz von 2 % Celite als Filterhilfsmittel filtriert. Es wird nach den folgenden Schemata verfahren:

Die Isolierung der erfindungsgemäßen Verbindungen der Formel I erfolgt nach Abtrennung der Biomasse aus dem Kulturfiltrat. Die Abtrennung wird beispielsweise durch Filtration oder Zentrifugation durchgeführt. Das Kulturfiltrat wird über ein Adsorberharz, z.B. auf Polystyrolbasis, gegeben.

Die Elution erfolgt mit einem polaren Lösungsmittel, bevorzugt eingesetzt werden niedere Alkohole, wie z.B. Methanol, die gegebenenfalls noch mit Wasser vermischt werden.

Die Verbindungen der Formel I können durch Chromatographie an Kieselgel mit Laufmittelsystemen, die Mischungen aus niederen Alkoholen, wie z.B. Methanol, Ethylacetat, n-Alkanen, wie z.B. Hexan oder Heptan, und chlorierten Kohlenwasserstoffen, wie z.B. Methylenchlorid, enthalten, isoliert werden. Auch reine Lösungsmittel sind verwendbar. Nachreinigungen können z.B. mittels HPLC an Kieselgel, Reversed Phase-Kieselgelen oder durch Gelpermeationschromatographie, wie z.B. an Sephadex-LH-20 in Laufmittelsystemen, die Mischungen aus niederen Alkoholen, wie z.B. Methanol, Ethylacetat, n-Alkanen, wie z.B. Hexan oder Heptan, und/oder chlorierten Kohlenwasserstoffen, wie z.B. Methylenchlorid, enthalten, oder auch in reinen Lösemitteln, durchgeführt werden.

Verbindungen der Formel I sind entweder viskose Flüssigkeiten oder farblose Feststoffe, welche mit Anisaldehyd-Schwefelsäure nach Erhitzen Farbreaktionen eingehen, wobei die Farbe von Dauer und Stärke des Erhitzens abhängt. Im allgemeinen erhält man bläulich bis grüne Farbreaktionen.

1. Charakterisierung der Verbindung SM 225 (Decarestrictin G)

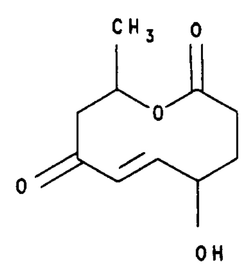

Dünnschichtchromatographie:

Kieselgel 60, $F_{254}$

a) n-Butanol/$CH_3COOH/H_2O$ = 4/1/5: $R_F$ 0.6

b) Chloroform/Methanol = 9/1: $R_F$ 0.74

c) Ethylacetat/n-Hexan = 3/1: $R_F$ 0.56

$^1$H-NMR-Spektrum (360 MHz, $CDCl_3$/TMS):

6.54 (1 H, m, J = 16 Hz, J = 7 Hz), 5.88 (1 H, br, d, J = 16 Hz), 5.06 (1H, m), 4.32 (1H, OH), 3.67 (1H,

m), 3.33 (1H, m), 1.91-2.01 (1H, m), 1.71-1.84 (2H, 2 m), 1.46-1.57 (1H, m), 1.22 (3H, d, J = 6.7 Hz)

$^{13}$C-NMR-Spektrum (90.5 MHz, $CDCl_3$/TMS):

195.5, 168.0, 150.5, 125, 68.0, 60.7, 53.5, 51.7, 33.0, 27.7

IR-Spektrum (Film): 3440, 2980, 2930, 2875, 1710, 1440, 1385, 1355, 1270, 1040, 1005, 980, 915, 760 $cm^{-1}$

Drehwert:$[\alpha]_D^{20}$ = + 14.49 (c = 0.207) in Methanol

EI-MS (70 eV): m/e = 198 (entsprechend $C_{10}H_{14}O_4$ oder 198.221)

Drehwert: $[\alpha]_D^{20}$ = + 14.49 (c = 0.163) in Methanol

2. Charakterisierung der Verbindung SM 281 (Decarestrictin M)

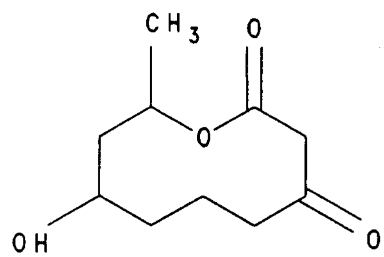

Dünnschichtchromatographie:

Kieselgel 60, $F_{254}$

a) n-Butanol/$CH_3COOH/H_2O$ = 4/1/5: $R_F$ 0.61

b) Chloroform/Methanol = 9/1: $R_F$ 0.77

c) Ethylacetat/n-Hexan = 3/1: $R_F$ 0.55

$^1$H-NMR-Spektrum (360 MHz, $CDCl_3$/TMS):

5.180 (m, 1H, J = 6.14, 11.02, 3.13), 3.68 (m, 1H, J = 9.41, 1.91), 3.41 (d, 1H, J = -15.0 Hz), 3.37 (d, 1H, J = -15.0 Hz), 2.71 (m, 1H, J = -14.8 Hz), 2.31 (m, 1H, J = -14.8 Hz); 2.04 (m, 1H), 2.01 (1 OH), 1.90 (m, 1H, J = 11.02, 9.41, -14.16 Hz), 1.87 (m, 1H, J = -14.16, 3.13, 1.91 Hz), 1.70 (m, 1H), 1.63 (m, 1H), 1.56 (m, 1H), 1.30 (d, 3H, J = 6.14)

$^{13}$C-NMR-Spektrum (90.5 MHz, $CDCl_3$/TMS):

203.04, 166.66, 71.67, 69.23, 51.71, 44.15, 39.35, 36.84, 21.32, 20.76

3. Charakterisierung der Verbindung SM 233 (Decarestrictin L)

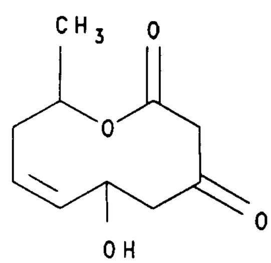

Dünnschichtchromatographie:

Kieselgel 60, $F_{254}$

a) n-Butanol/$CH_3COOH/H_2O$ = 4/1/5: $R_F$ 0.63

b) Chloroform/Methanol = 9/1: $R_F$ 0.76

c) Ethylacetat/n-Hexan = 3/1: $R_F$ 0.69

$^1$H-NMR-Spektrum (200 MHz, $CDCl_3$/TMS):

5.60-5.75 (2H, 2m), 5.13 (1H, ddq, J = 5 Hz, J = 2 Hz, J = 6.8 Hz), 4.77 (1H, ddd, J = 5 Hz, J = 8 Hz, J = 9 Hz), 3.48 (1H, m), 3.47 (1H, m), 2.97 (1H, dd, J = 12,5 Hz, J = 5 Hz), 2.91 (1H, ddd, J = 4 Hz, J = 5Hz, J = 14,2 Hz), 2.83 (1H, dd, J = 9 Hz, J = 12.5 Hz), 2.03 (1H, ddd, J = 4 Hz, J = 6.5 Hz, J = 14.2 Hz), 1.94 (1H, OH), 1.23 (3H, d, J = 6.8 Hz)

$^{13}$C-NMR-Spektrum (50.3 MHz, $CDCl_3$/TMS):

198.56, 165.69, 136.43, 124.61, 69.72, 63.63, 53.49, 51.38, 30.49, 17.74

4. Charakterisierung der Verbindung SM 140E (Decarestrictin E)

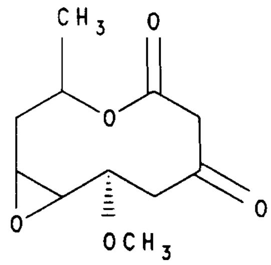

Dünnschichtchromatographie:
Kieselgel 60, $F_{254}$
a) n-Butanol/$CH_3COOH/H_2O$ = 4/1/5: $R_F$ 0.55
b) Chloroform/Methanol = 9/1: $R_F$ 0.77
c) Ethylacetat/n-Hexan = 3/1: $R_F$ 0.60
$^1$H-NMR-Spektrum (360 MHz, $CDCl_3$/TMS):
5.11 (m, 1H, J = 1.19 Hz, J = 11.45 Hz, J = 6.33 Hz), 3.50 (3H, s),3.47 (m, 1H), 3.44 (m, 1H), 3.29 (1H, ddd, J = 9,09 Hz, J = 3.13 Hz, J = 4.47 Hz), 3.05 (1H, m, J = 4.06 Hz, J = 10.35 Hz, J = 4.26 Hz), 2.98 (m, 1H), 2.90 (1H, dd, J = 4.47 Hz, J = 14.13 Hz), 2.63 (1H, dd), 2.35 (m, 1H), 1.54 (m, 1H), 1.33 (3H, q, J = 6.33 Hz)
$^{13}$C-NMR-Spektrum (90.5 MHz, $CDCl_3$/TMS):
200.31, 165.41, 77.34, 68.98, 59.58, 57.43, 53.19, 52.11, 46.22, 36.70, 20.64
EI-GC/MS (70 eV), GC/CI-$NH_3$ (Trennsäule HP 5,25 m*0,2 mm, 50 - 320 ° C) m/e = 288 ($M^+$)
5. Chakterisierung der Verbindung SM 230 (Decarestrictin I)

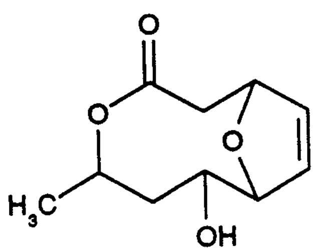

Dünnschichtchromatographie:
Kieselgel 60, $F_{254}$
a) n-Butanol/$CH_3COOH/H_2O$ = 4/1/5: $R_F$ 0.37
b) Chloroform/Methanol = 9/1: $R_F$ 0.77
c) Ethylacetat/n-Hexan = 3/1: $R_F$ 0.60
$^1$H-NMR-Spektrum (200 MHz, $CDCl_3$/TMS):
5.94 (m, 2H), 5.11 (m, 2H), 4.98 (m, 1H), 3.97 (dt, 1H, J = 10.8 und 2 Hz), 2.70 (m, 2H), 2.42 (1H, OH), 2.02 (m, 1H), 1.69 (m, 1H), 1.29 (d, 3H, J = 13 Hz)
$^{13}$C-NMR-Spektrum (90.5 MHz, $CD_3OD$/TMS):
173.00, 133.03, 128.06, 93.49, 82.69, 74.42, 72.21, 43.20, 40.37, 21.89
IR-Spektrum (KBr):
3405, 2980, 2860, 1710, 1440, 1380, 1297, 1252, 1163, 1070, 980, 705 $cm^{-1}$
UV-Spektrum (MeOH): Endabsorption
MS: m/e $C_{10}H_{14}O_4$ 198.22 gef.: $M^+$-CO
Drehwert: $[\alpha]_D^{20}$: -132.2 °

| Elementaranalyse | gef.: C 60.2 %<br>gef.: H 7,1 % | ber.: C 60.6 %<br>ber.: H 7,1 % |
|---|---|---|

Eine antibakterielle Wirkung kann durch übliche Hemmhoftests in vitro gezeigt werden. Die Verbindungen der Formel I zeigen besondes bei Staphylococcus aureus gute Wirkung. Die maximale Hemmkonzentration liegt im Bereich von > 10 bis < 100 µg/ml.

**Patentansprüche**

**1.** Verbindungen der Formel I,

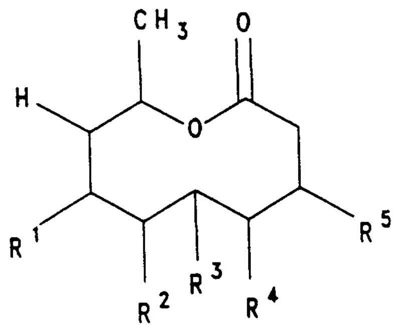

in der

$R^1$ Hydroxyl, Oxo oder zusammen mit $R^2$ eine Doppelbindung oder cyclisch mit Sauerstoff verknüpft ist, im letzteren Falle dann aber $R^3$ für Methoxy steht,
$R^2$ Wasserstoff oder zusammen mit $R^5$ cyclisch mit einem Sauerstoff verknüpft oder zusammen mit $R^3$ eine Doppelbindung bedeutet, im letzteren Falle dann aber $R^1$ für Oxo steht,
$R^3$ Wasserstoff, Hydroxy oder Methoxy oder zusammen mit $R^4$ eine Doppelbindung bedeutet,
$R^4$ Wasserstoff oder Hydroxy und
$R^5$ Wasserstoff oder Oxo bedeuten.

**2.** Verfahren zur Herstellung der Verbindungen der Formel I nach Anspruch 1, dadurch gekennzeichnet, daß Penicillium sp. in einem Nährmedium kultiviert wird, bis sich die Verbindung der allgemeinen Formel I in der Kultur anhäuft.

**3.** Verfahren nach Anspruch 2, dadurch gekennzeichnet, daß die Stämme DSM 4209 und/oder DSM 4210 kultiviert werden.

**4.** Verfahren nach den Ansprüchen 2 und 3, dadurch gekennzeichnet, daß das Nährmedium 0,2 bis 5 % Malzextrakt, 0,02 bis 0,5 % Hefeextrakt, 0,1 bis 5 % Glucose sowie 0,005 bis 0,2 % Ammoniumsalz enthält, jeweils bezogen auf das Gewicht der gesamten Nährlösung.

**5.** Verfahren nach Anspruch 4, dadurch gekennzeichnet, daß das Nährmedium 1 bis 4 % Malzextrakt, 0,1 bis 0,4 % Hefeextrakt, 0,5 bis 2 % Glucose und 0,01 bis 0,1 % Ammoniumsalz enthält.

**6.** Verfahren nach einem oder mehreren der Ansprüche 2 bis 5, dadurch gekennzeichnet, daß die Kultivierung bei 18 bis 35 °C erfolgt.

**7.** Verfahren nach einem oder mehreren der Ansprüche 2 bis 6, dadurch gekennzeichnet, daß die Kultivierung in dem pH-Bereich zwischen 1 und 8 erfolgt.

**8.** Verbindung der Formel I nach Anspruch 1 zur Anwendung in einem therapeutischen Verfahren.

**9.** Verwendung einer Verbindung nach Anspruch 1 zur Herstellung eines antibakteriellen und lipidregulatorischen Arzneimittels.

**Patentansprüche für folgenden Vertragsstaat : ES**

1. Verfahren zur Herstellung der Verbindungen der Formel I

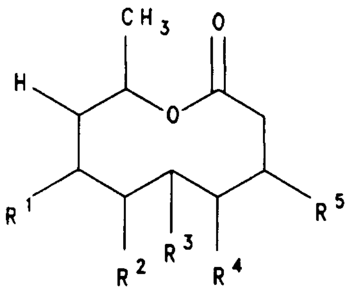

in der

$R^1$ Hydroxyl, Oxo oder zusammen mit $R^2$ eine Doppelbindung oder cyclisch mit Sauerstoff verknüpft ist, im letzteren Falle dann aber $R^3$ für Methoxy steht,
$R^2$ Wasserstoff oder zusammen mit $R^5$ cyclisch mit einem Sauerstoff verknüpft oder zusammen mit $R^3$ eine Doppelbindung bedeutet, im letzteren Falle dann aber $R^1$ für Oxo steht,
$R^3$ Wasserstoff, Hydroxy oder Methoxy oder zusammen mit $R^4$ eine Doppelbindung bedeutet,
$R^4$ Wasserstoff oder Hydroxy und
$R^5$ Wasserstoff oder Oxo bedeuten,

dadurch gekennzeichnet, daß Penicillium sp. in einem Nährmedium kultiviert wird, bis sich die VErbindung der allgemeinen Formel I in der Kultur anhäuft.

2. Verfahren nach Anspruch 1, dadurch gekennzeichnet, daß Penicillium sp. bei einer Temperaturvon 18 - 35 ° C, in einem pH-Bereich von 1 - 8 und über einen Zeitraum von 60 - 170 Stunden kultiviert wird.

3. Verfahren nach Anspruch 1 oder 2, dadurch gekennzeichnet, daß die Stämme DSM 4209 und/oder DSM 4210 kultiviert werden.

4. Verfahren nach einem oder mehreren der obengenannten Ansprüche, dadurch gekennzeichnet, daß das Nährmedium 0,2 bis 5 % Malzextrakt, 0,02 bis 0,5 % Hefeextrakt, 0,1 bis 5 % Glucose sowie 0,005 bis 0,2 % Ammoniumsalz enthält, jeweils bezogen auf das Gewicht der gesamten Nährlösung.

5. Verfahren nach Anspruch 4, dadurch gekennzeichnet, daß das Nährmedium 1 bis 4 % Malzextrakt, 0,1 bis 0,4 % Hefeextrakt, 0,5 bis 2 % Glucose und 0,01 bis 0,1 % Ammoniumsalz enthält.

6. Verfahren nach einem oder mehreren der Ansprüche 2 bis 5, dadurch gekennzeichnet, daß die Kultivierung bei 25 bis 30 ° C erfolgt.

7. Verfahren nach einem oder mehreren der Ansprüche 2 bis 6, dadurch gekennzeichnet, daß die Kultivierung in dem pH-Bereich zwischen 1 und 4 erfolgt.

8. Verfahren zur Herstellung der Verbindungen der Formel I nach Anspruch 1 zur Anwendung in einem therapeutischen Verfahren.

9. Verwendung einer Verbindung nach Anspruch 1 zur Herstellung eines antibakteriellen und lipidregulatorischen Arzneimittels.

Europäisches Patentamt

# EUROPÄISCHER RECHERCHENBERICHT

Nummer der Anmeldung

EP 92 10 1443

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | CHEMICAL ABSTRACTS, vol. 115, no. 15, 14. Oktober 1991, Columbus, Ohio, US; abstract no. 128938, J.R. MAHAJAN et al.: 'Alternative syntheses of phoracantholide I (9-decanolide)' Seite 918 ; & JOURNAL OF THE BRAZILIAN CHEMICAL SOCIETY, 1990, 1(3), 119-123 * Zusammenfassung und Verbindung mit CAS RN 136035-54-6 * | 1 | C07D313/00<br>C07D493/04<br>C07D493/08<br>A61K31/365<br>C12P17/08<br>C12P17/18 |
| D,A | EP-A-0 333 024 (HOECHST) * das ganze Dokument * | 1,2,8,9 | |
| | | | **RECHERCHIERTE SACHGEBIETE (Int. Cl.5)** |
| | | | C07D |

Der vorliegende Recherchenbericht wurde für alle Patentansprüche erstellt

| Recherchenort | Abschlußdatum der Recherche | Prüfer |
|---|---|---|
| DEN HAAG | 24 MAERZ 1992 | RUSSELL F. ENGLISH |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)